# EUROPEAN PATENT APPLICATION

(11) **EP 4 393 489 A1**
(43) Date of publication of application: **03.07.2024**
(21) Application number: 22849946.3
(22) Date of filing: 29.07.2022
(51) Int. Cl.: A61K 31/166, A61K 31/44, A61K 31/416, A61K 31/381, A61K 31/18, A61K 31/5377, A61K 31/197, A61K 31/222, A61P 37/00, A61P 37/06

(54) **NOVEL BENZENE DERIVATIVE AND IMMUNOSUPPRESSION-RELATED USE THEREOF**

(30) Priority: 29.07.2021 KR 20210100261
(71) Applicant: Provibio Co., Ltd., Gimhae-si, Gyeongsangnam-do 50834 (KR)
(72) Inventor: PARK, Seok Ju, Busan 48063 (KR)
(74) Representative: Rückerl, Florian
(86) International application number: PCT/KR2022/011254
(87) International publication number: WO 2023/008973

(57) **Abstract**

The present invention relates to a novel benzene derivative and immunosuppressive-related use thereof. Specifically, it relates to a composition comprising a compound of Formula 1, its stereoisomer, solvate, hydrate, crystalline form, or pharmaceutically acceptable salt. The composition has preventive or therapeutic applications in immune disorders such as autoimmune diseases, transplant rejections, allergic diseases, and inflammatory conditions.

## Description

### [Technical Field]

The present invention relates to a novel benzene derivative and immunosuppressive-related use thereof. Specifically, the present invention relates to a composition comprising a compound of Formula 1, a stereoisomer, a solvate, a hydrate, a crystalline form, or a pharmaceutically acceptable salt thereof. The composition is used for preventing or treating immune disorders such as autoimmune disease, transplant rejection reaction, allergic disease, or inflammatory disease.

### [Background Art]

Autoimmunity refers to an inappropriate response to autoantigens in the immune system, resulting in damage to cells or tissues by either humoral or cellular immunity, or both. Autoimmune disease is related to a molecule, cell, and tissue targeted by autoimmune responses, and they may manifest systemically depending on the distribution of target antigens or specifically in certain organs. For example, systemic lupus erythematosus (SLE), rheumatoid arthritis (RA), and multiple sclerosis (MS) belong to systemic autoimmune disease, while autoimmune hemolytic anemia, insulin-dependent diabetes mellitus (IDDM), and Graves' disease belong to organ-specific autoimmune disease.

Transplant rejection occurs when transplanted tissues are rejected by the immune system of the recipient, resulting in the destruction of the transplanted tissues. This may be classified into hyperacute rejection, acute rejection, and chronic rejection.

Allergic diseases refer to allergy symptoms that occur when the immune system overreacts to specific substances, known as allergens, upon exposure or contact, causing inflammation in the affected area. It includes bronchial asthma, allergic rhinitis, atopic dermatitis, hives (such as angioedema, rash), anaphylactic reaction, allergic conjunctivitis, seasonal allergies, drug allergies, food allergies, etc.

Inflammatory disease refers to abnormal inflammation caused by physical factors such as burns and trauma, biological factors such as pathogens, hypersensitivity and stress, and various chemical factors such as toxins and fine dust.

Autoimmune diseases, transplant rejections, allergic diseases, and inflammatory diseases have a common characteristic of being triggered by excessive immune response. Such immune disorders affect a large number of patients, and a research and development for treating or preventing those has been conducted for a long time. For example, antihistamines and steroid agents have been developed and are still commonly used. However, due to problems such as toxicity and resistance, there is still a need for substances that can provide desired therapeutic or preventive effects without side effects or adverse reactions in the art.

### [Technical Problem]

The inventors have recognized the problems with the conventional techniques and have conducted numerous trials and research to discover compounds that offer excellent therapeutic, preventive, or ameliorative effects for immune disorders selected from autoimmune diseases, transplant rejections, allergic diseases, and inflammatory diseases. As a result, the compound of Formula 1 was developed, leading to the completion of the present invention.

### [Technical Solution]

The compound according to the present invention, aimed at achieving the objectives of the invention, is represented by the following Formula 1: 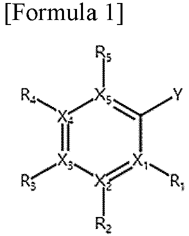 in the Formula 1,
- among X₁, X₂, X₃, X₄ and X₅, one or two atoms are nitrogen, and the other atoms are carbon,
- each R₁, R₂, R₃, R₄ and R₅ is independently selected among hydrogen, halogen, C1-C14 alkyl, C2-C14 alkenyl, C2-C14 alkynyl, C5-C14 aryl, C5-C14 arylalkyl, C3-C14 cycloalkyl, C3-C14 cycloalkylalkyl, C3-C14 heteroaryl, C3-C14 heteroarylalkyl, C3-C14 heterocycloalkyl, C3-C14 heterocycloalkylalkyl, C1-C14 alkoxy, C5-C14 aryloxy, C5-C14 heteroaryloxy, -NO₂, -NR₆, -SO₂R₆, -SO₂N(R₆)₂, -CF₃, -OH, cyanide, thiol, triazine, triazole, tetrazole, thiazole, diazole, imidazole, alkylamine, cycloalkylamine, heterocycloamine, and biotin,
- Y is selected among -OH, thiol, phenoxy, cyanide, thiazole, diazole, imidazole, triazine, triazole, tetrazole, -CON(R₆)₂, -NR₆COR₆, -SO₂N(R₆)₂, -NH₂, -NO₂, -COOR₆, -R₇COOR₆, -R₇CON(R₆)₂, -CONHSR₆, -CONHR₇SR₆, -CONHSSR₆, -CONHR₇SSR₆, - CONHR₇SO₂R₆, -CONHOR₆, -CONHR₇OR₆, -CON(R₇)₂CON(R₆)₂, and - CON(R₇)₂NR₇COR₆,
   wherein each R₆ and R₇ is independently selected among hydrogen, C1-C14 alkyl, C2-C14 alkenyl, C2-C14 alkynyl, C6-C12 aryl, C6-C12 arylalkyl, C3-C12 cycloalkyl, C3-C12 cycloalkylalkyl, C3-10 heteroaryl, C3-10 heteroarylalkyl, C3-10 heterocycloalkyl, C3-10 heterocycloalkylalkyl, alkylamine, cycloalkylamine, heterocycloamine and biotin, unsubstituted or substituted with one or more of hydrogen, halogen, thiol, phenoxy, cyanide, thiazole, diazole, imidazole, triazine, triazole, tetrazole, C1-C6 alkyl, C2-C6 alkenyl, C2-C6 alkynyl, C6-C12 aryl, C6-C12 arylalkyl, C3-C12 cycloalkyl, C3-C12 cycloalkylalkyl, C3-10 heteroaryl, C3-10 heteroarylalkyl, C3-10 heterocycloalkyl, C3-10 heterocycloalkylalkyl, C1-C6 alkoxy, -CF₃, amine, C1-6 alkylamine, and -CN.

The Formula 1 may be used interchangeably with the chemical Formula and has the same meaning.

The term "independently" used herein refers to that two or more substituents may be individually defined and may be different from or the same as each other. For example, X₁, X₂, X₃, X₄, X₅ and X₆ are carbon or nitrogen and may be the same or different.

The terms "alkyl, alkenyl, alkynyl" used herein include both linear or branched forms.

The term "halogen" used herein refers to fluorine, chlorine, bromine, or iodine.

The term "alkoxy" used herein refers to O-alkyl.

The term "hetero" used herein refers to heteroatoms selected from oxygen, nitrogen and sulfur.

The term "heterocycloalkyl" used herein refers to cycloalkyl group containing heteroatom within the ring.

The present invention provides a composition comprising the compound represented by Formula 1, its stereoisomer, a solvate, or a pharmaceutically acceptable salt thereof for treating or preventing immune disorder selected from autoimmune disease, transplant rejection reaction, allergic disease, and inflammatory disease.

The term "treatment" or "treating" used herein refers to any action in which symptom of immune disorder is improved or cured by administration of the composition of the present invention.

The term "prevention" or "preventing" used herein refers to any action in which symptom of immune disorder is suppressed or delayed by administration of the composition of the present invention.

The aforementioned immune disorder should be understood to include symptoms, conditions, or diseases that progress further or result in increased severity of immune disorder.

For example, autoimmune disease may include, but not limited to, systemic lupus erythematosus (SLE), rheumatoid arthritis (RA), multiple sclerosis (MS), systemic sclerosis, autoimmune hemolytic anemia, insulin-dependent diabetes mellitus (IDDM), type 1 diabetes, Graves' disease, Graves hyperthyroidism, Kikuchi disease, Hemophagocytic lymphohistiocytosis, Adult onset Still's disease, Behcet's disease, IgG4-related diseases, and psoriasis.

Allergic disease may include, but not limited to, bronchial asthma, allergic rhinitis, atopic dermatitis, hives (angioedema, rashes etc.), anaphylaxis reaction, allergic conjunctivitis, seasonal allergy, drug allergy, and food allergy.

Inflammatory disease may include, but are not limited to, inflammation disorder caused by physical factors such as burn, injury; biological factors such as pathogens, hypersensitivity, stress; or various chemical factors such as toxins, dust.

The aforementioned compound includes its stereoisomer, solvate, hydrate, crystalline form, or pharmaceutically acceptable salt.

The term "pharmaceutically acceptable salt" as used herein refers to a salt that may be prepared by a conventional method known in the art, for example, but not limited to, a pharmaceutically acceptable salt of the acids formed by reacting with inorganic acids such as hydrochloric acid, hydrogen bromide, sulfuric acid, sodium hydrogen sulfate, phosphoric acid, carbonic acid, etc., or with organic acids such as formic acid, acetic acid, oxalic acid, benzoic acid, citric acid, tartaric acid, gluconic acid, gestisic acid, fumaric acid, lactobionic acid, salicylic acid or acetylsalicylic acid (aspirin); or a pharmaceutically acceptable metal salt formed by reacting with alkali metal ions such as sodium and potassium; or a pharmaceutically acceptable salt formed by reacting with ammonium ions.

The pharmaceutical composition of the present invention may comprise a compound of Formula 1, its stereoisomer, solvate, hydrate, crystalline form, or pharmaceutically acceptable salt alone, or further comprise one or more pharmaceutically acceptable carriers, excipients, or diluents.

The pharmaceutically acceptable carrier may additionally include, for example, a carrier for oral administration or non-oral administration. The carrier for oral administration may include lactose, starch, cellulose derivative, magnesium stearate, stearic acid, etc. The carrier for non-oral administration may include water, suitable oil, saline, aqueous glucose, and glycol, etc, and may further include stabilizer and preservative. The stabilizer may include antioxidant such as sodium bisulfite, sodium sulfite, or ascorbic acid. The suitable preserving agent may include benzalkonium chloride, methyl- or propyl-paraben, or chlorobutanol. Other pharmaceutically acceptable carriers may be those disclosed in the literature "Remington's Pharmaceutical Sciences, 19th ed., Mack Publishing Company, Easton, PA, 1995".

The pharmaceutical composition of the present invention may be administered to mammals including human using various methods. For example, it may be administered orally or parenterally. The parenteral administration may include, but are not limited to, intravenous, intramuscular, intraarterial, intramarrow, intradural, intracardial, percutaneous, subcutaneous, intraperitoneal, intranasal, intraintestinal, topical, sublingual, rectal administration, etc. As an example, the pharmaceutical composition of the present invention may be administered by lightly pricking the skin with a 30-gauge fine needle after preparing it in injectable form, or by directly applying it to the skin.

The pharmaceutical composition according to the present invention may be formulated as a formulation for oral administration or a formulation for parenteral administration.

The formulation for oral administration may be prepared in the form of powders, granules, tablets, pills, sugar-coated pills, capsules, liquids, gels, syrups, slurries, suspensions, etc., using methods known in the art. For example, the active ingredient of the present invention may be mixed with suitable excipient(s) and/or adjuvant(s), grinding it and then processed into a granule mixture to obtain a tablet or a sugar-coated tablet for oral administration. Examples of suitable excipient may include, but are not limited to, sugars including lactose, dextrose, sucrose, sorbitol, mannitol, xylitol, erythritol, maltitol, etc., starches including corn starch, wheat starch, rice starch, potato starch, etc., celluloses including cellulose, methyl cellulose, sodium carboxymethylcellulose, hydroxypropylmethylcellulose, etc., and fillers such as gelatin, polyvinyl pyrrolidone, etc. Optionally, disintegrating agents such as crosslinked polyvinyl pyrrolidone, agar, alginic acid or sodium alginate may be added. Further, the pharmaceutical composition of the present invention may further comprise anticoagulants, lubricants, wetting agents, aromatic agents, emulsifiers and preservatives, etc.

The formulation for parenteral administration may be prepared in the form of injections, creams, lotions, topical ointments, oils, moisturizers, gels, aerosols, nasal inhalers using methods known in the art. These formulation may be those disclosed in the literature "Remington's Pharmaceutical Science, 15th Edition, 1975. Mack Publishing Company, Easton, Pennsylvania 18042, Chapter 87: Blaug, Seymour".

The total effective dose of the pharmaceutical composition according to the present invention may be administered to a subject in a single dose, or in multiple doses through a fractionated treatment protocol. The pharmaceutical composition may vary the concentration of active ingredients according to the symptom of the disease. For example, the total dose of the pharmaceutical composition according to the present invention may preferably be about 0.01µg to 1,000mg per 1kg body weight of a patient per day, or the most preferably 0.1µg to 100mg. The appropriate dose of the pharmaceutical composition according to the present invention or the contents of active ingredient in the pharmaceutical composition may be determined considering various factors such as administration route, times administered, patient age, body weight, health, gender, severity of disease, diet and excretion rate, etc. by a person having ordinary skill in the art. There is no particular limit to the dosage form, administration route and administration method, so long as the pharmaceutical composition shows the effect of the invention.

Furthermore, the pharmaceutical composition of the present invention may be administered as an individual therapeutic agent or may be administered in combination with other therapeutic agents. When administered in combination with other therapeutic agent, the composition of the present invention and the other therapeutic agents may be administered concurrently, individually, or sequentially. The therapeutic agent may include substances already known to have therapeutic or ameliorative effects on immune disorder. The other therapeutic agent include not only pharmacotherapy but also surgical interventions, procedures, and surgeries.

When the pharmaceutical composition of the present invention is administered in combination with other therapeutic agents, the composition of the present invention and the other therapeutic agents may be separately formulated into individual containers or may be co-formulated in the same container.

In another aspect, the present invention provides a method for treating or preventing immune disorder administering the composition of the present invention to a subject.

In the method for treating or preventing immune disorders according to the present invention, each term has the same meaning as described unless specifically mentioned otherwise.

The term "subject" as used herein may include a human or any non-human animal. The term "non-human animal" may be a vertebrate, such as a non-human primate, sheep, dog, cow, horse, pig, rodent, such as mouse, rat, and guinea pig. In this specification, the "subject" may be used interchangeably with "individual" and "patient".

In the method for treating or preventing immune disorder according to the present invention, the composition of the present invention may be administered sequentially, simultaneously, or separately with other therapeutic agents. The "sequential" administration means that the composition of the present invention and other therapeutic agents are administered relatively continuously, allowing the minimum possible time as the time consumed in the administration interval. The "individual" administration means that the composition of the present invention and other therapeutic agents are administered at regular intervals. The administration method of the composition of the present invention and/or the other therapeutic agents may be appropriately selected by those skilled in the art considering therapeutic effects and side effects of the patient.

The composition comprising compound of Formula 1, its stereoisomer, solvate, hydrate, crystalline form, or pharmaceutically acceptable salt can effectively prevent or treat immune disorders selected from autoimmune disease, transplant rejection reaction, allergic disease, and inflammatory condition.

### [Brief Description of Drawings]

FIG. 1 is a FACS graph to explain the relationship between CFSE fluorescence staining and cell proliferation.
FIG. 2 is a graph showing cell proliferation read by FACS after administering each compound of the present invention.
FIG. 3 to FIG. 6 are graphs measuring the secretion levels of IL-2 after 24 hours, and of IFN-γ, TNF-α, and IL-17A after 72 hours, respectively, following drug treatment.
FIG. 7 to FIG. 9 show the methods and results for evaluating the efficacy of the compounds of the present invention in a psoriasis mouse animal model.
FIG. 10a to FIG. 10c are graphs depicting the results of evaluating the efficacy of the compounds of the present invention in a psoriasis mouse animal model, specifically regarding the ratio of CD1 1b granulocytes, B cells, and T cells.

### [Examples]

Hereinafter, examples are presented to help the understanding of the present invention. However, the following examples are provided for easier understanding of the present invention, and the scope of the present invention is not limited by the following examples. Those having ordinary skill in the art may make various modifications to the present invention within the scope not departing from the inventive concept. Terms that are not specifically defined in this specification should be understood to have meanings commonly used in the technical field to which the present invention pertains.

### Preparation Example

Compounds belongnig to Formula 1 were prepared, for example, according to Scheme 1 or 2 below. Compounds having different substituents were prepared through similar steps, but not all examples are described here. Referring to the following representative examples, those skilled in the art are able to easily prepare compounds of Formula 1.

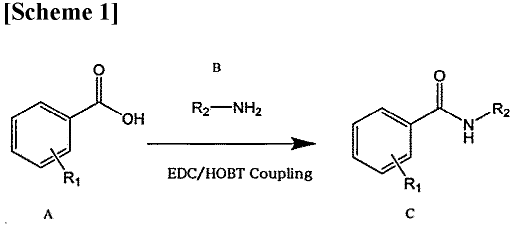

In the Scheme 1, Compound C is obtained by coupling reaction from Compound A and Compound B, using 1-ethyl-3-(3'-dimethylaminopropyl)carbodiimide hydrochloride (EDC•HCl) and hydroxybenzotriazole (HOBt).

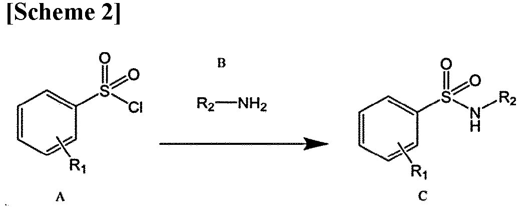

In the Scheme 2, Compound C is obtained from Compound A and Compound B.

Representative examples of compounds prepared in this manner are provided below along with their specific chemical structure. It is noted that all compounds shown in the table are not comprised in Formula 1 according to the present invention.

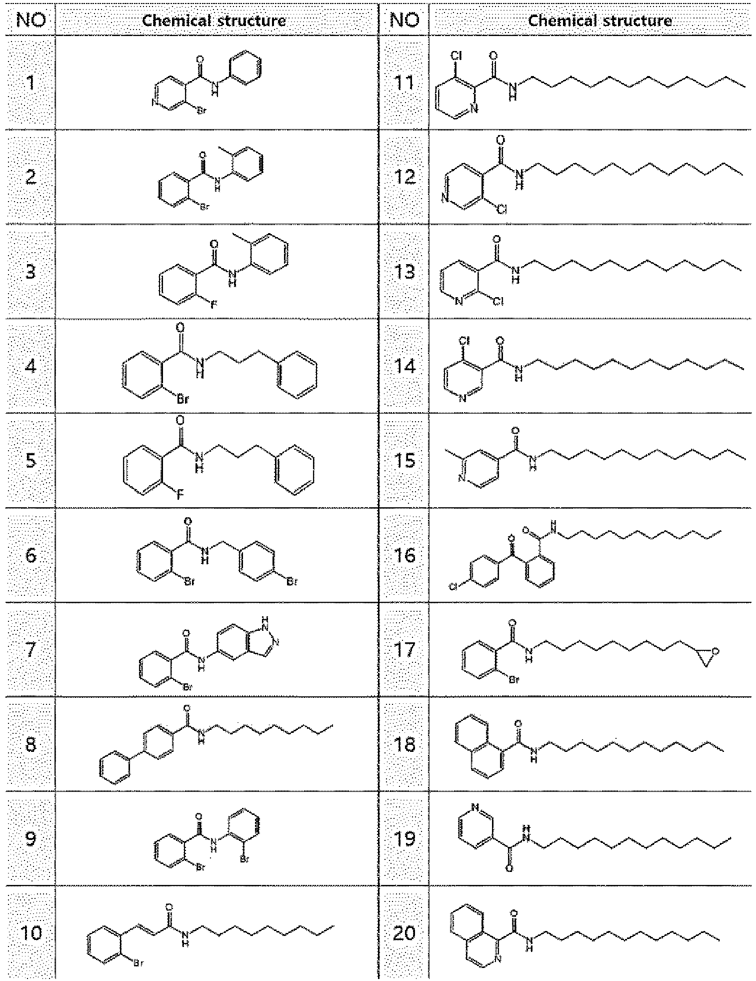

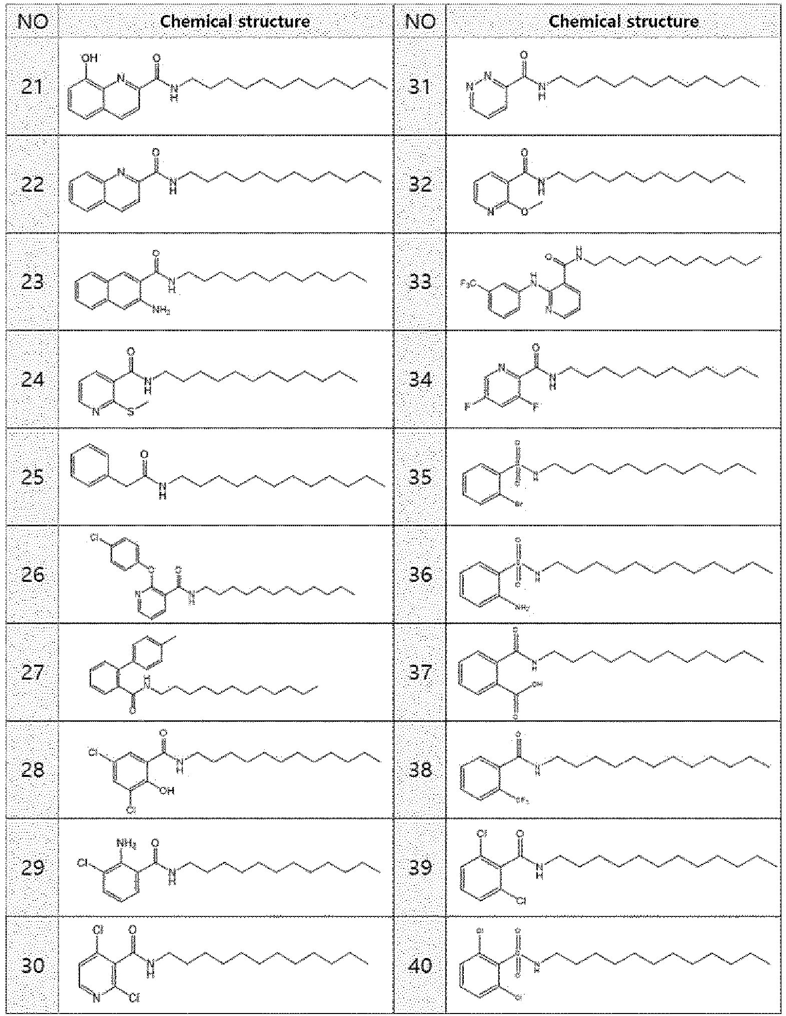

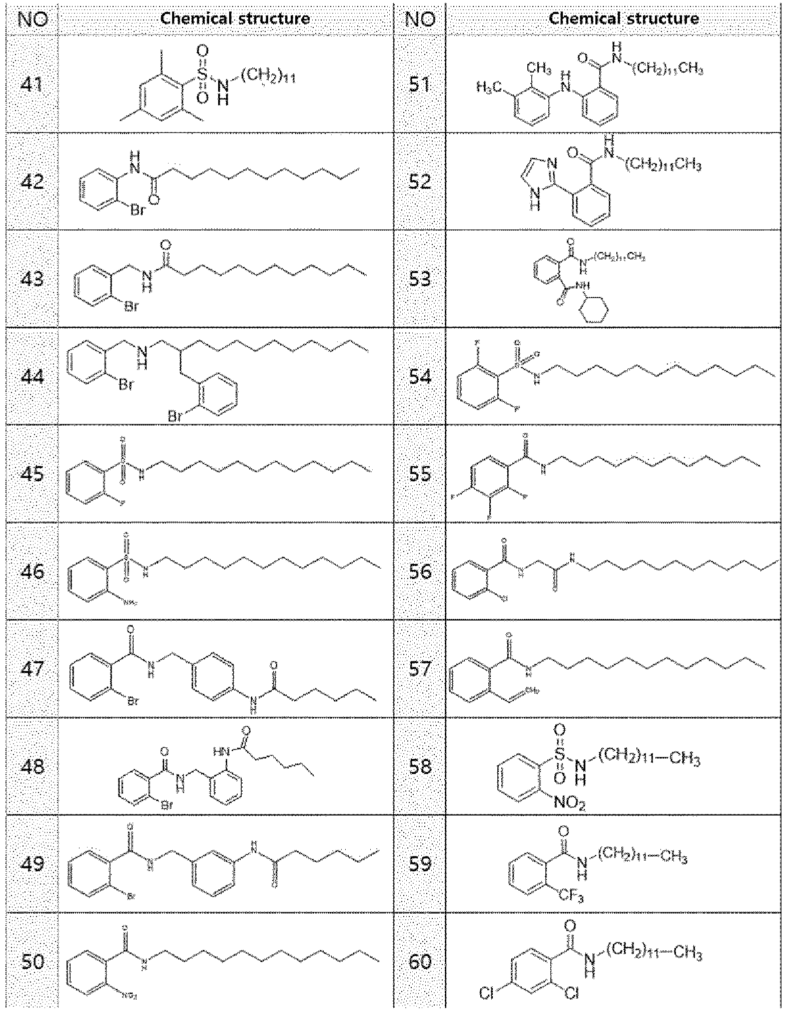

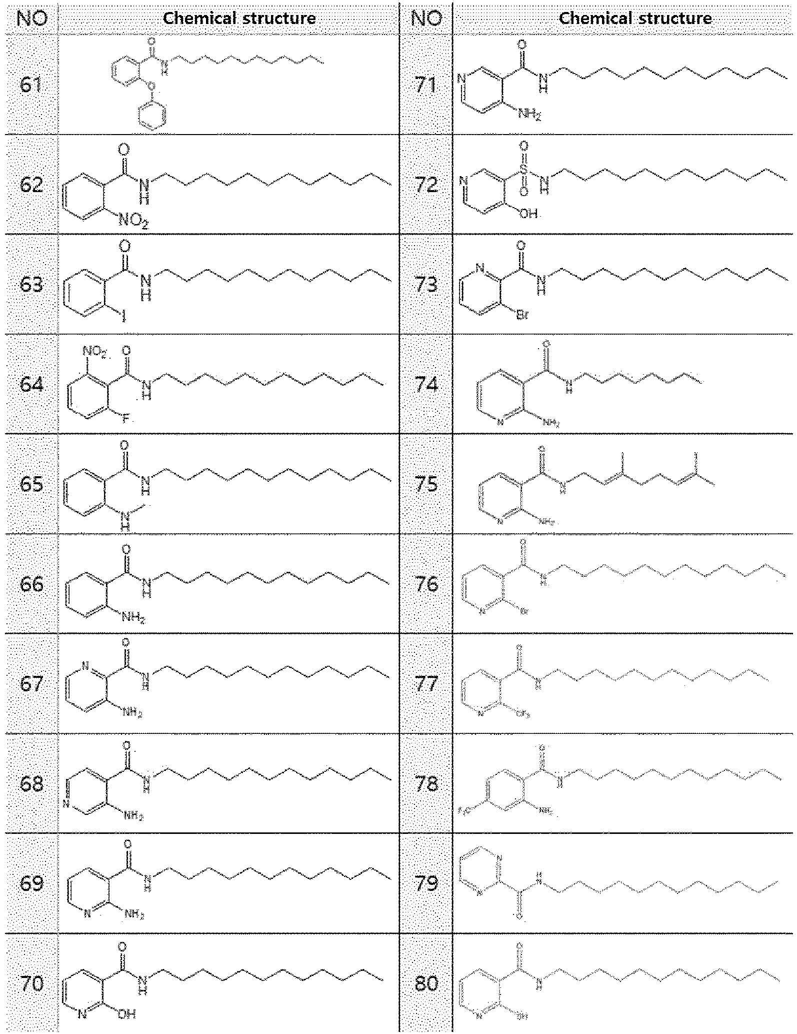

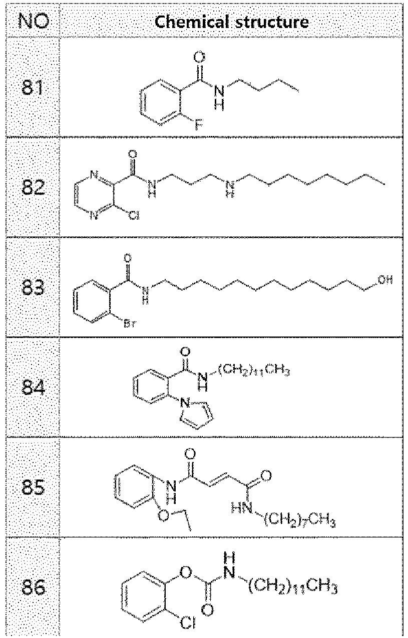

After preparing the compounds, the measured molecular weights, properties, and 1H NMR data thereof are as in Table 1 below.

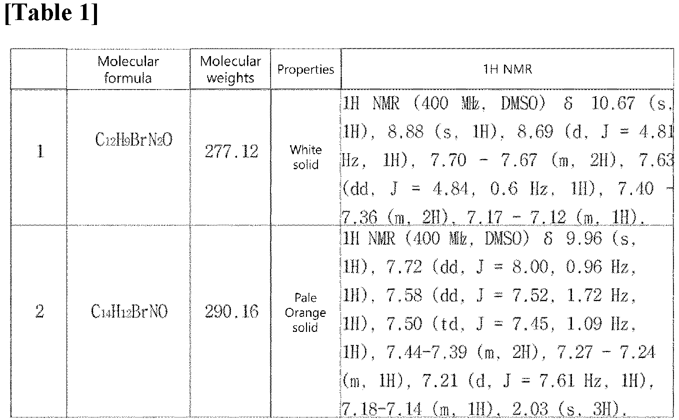

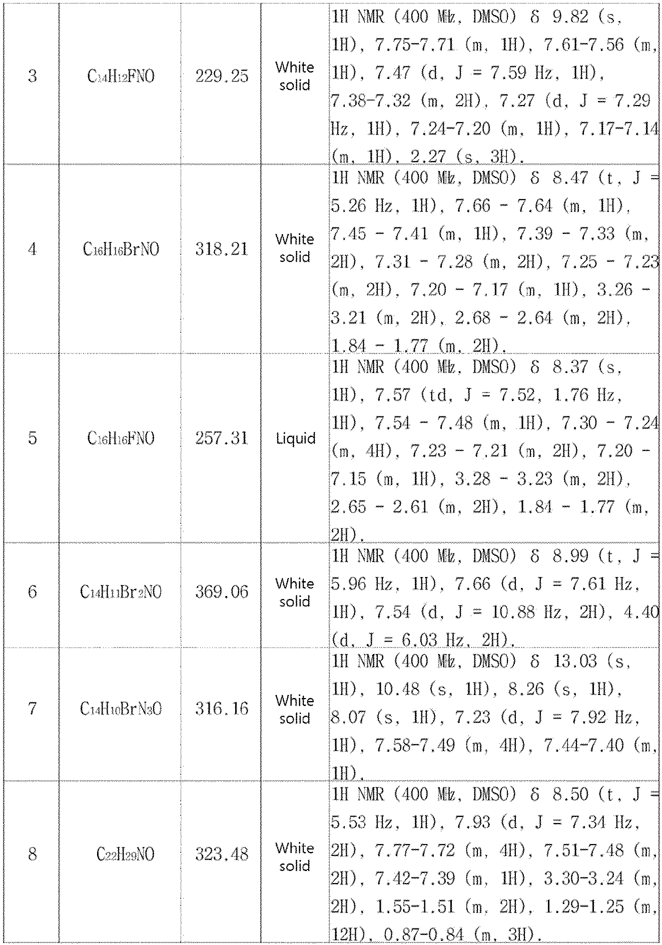

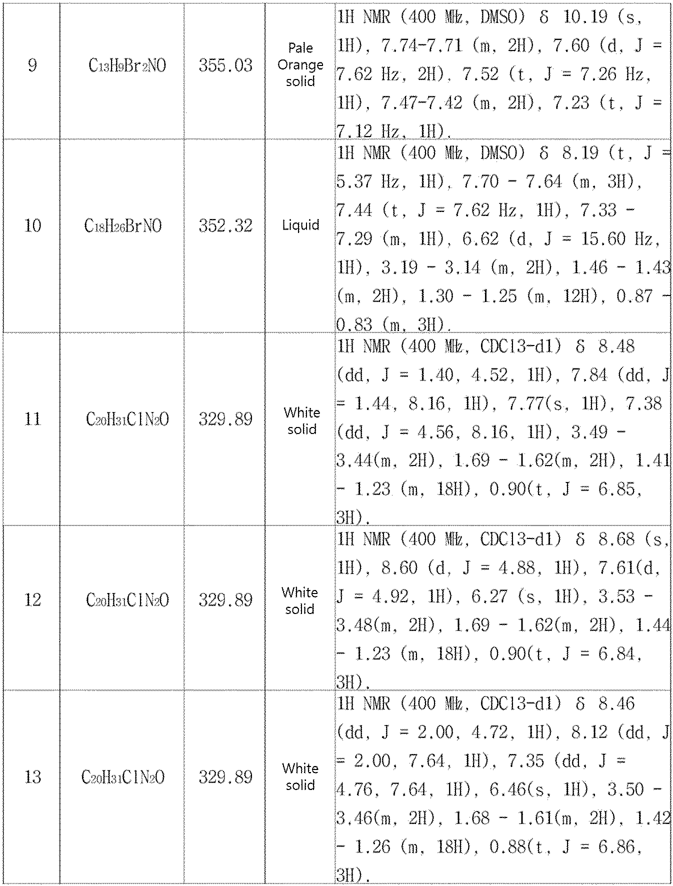

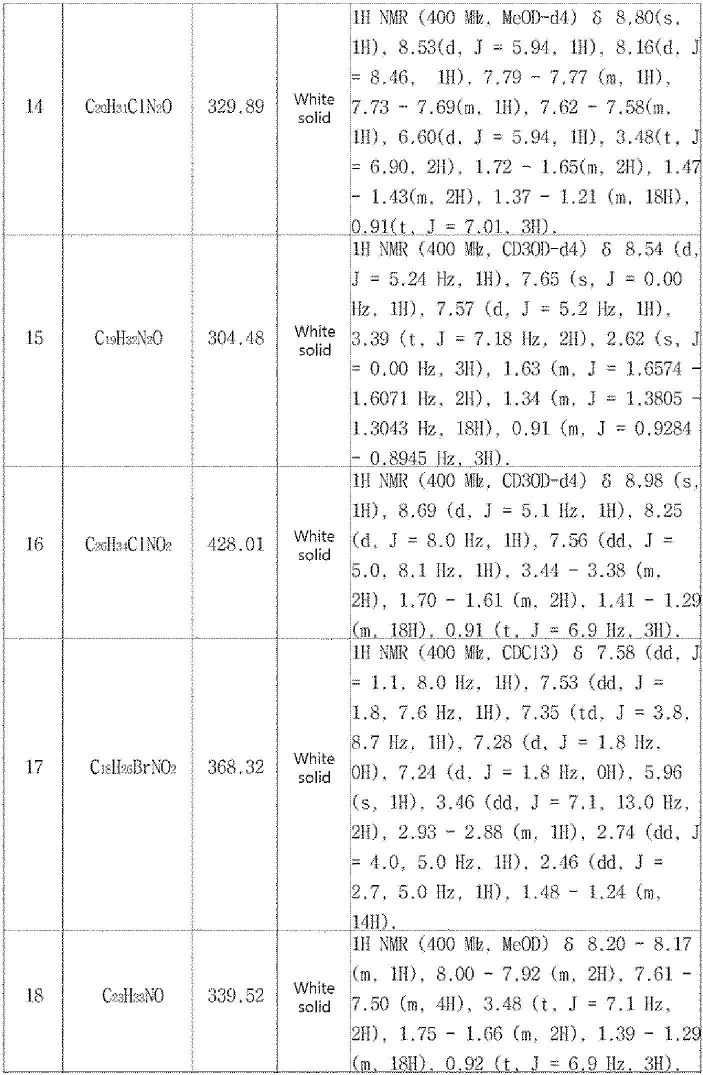

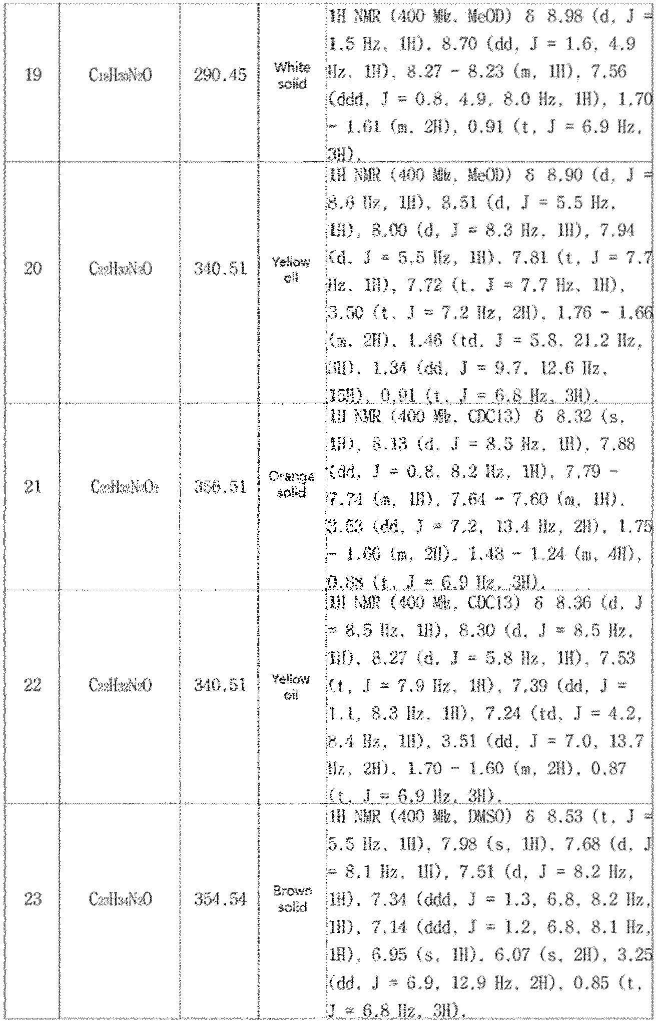

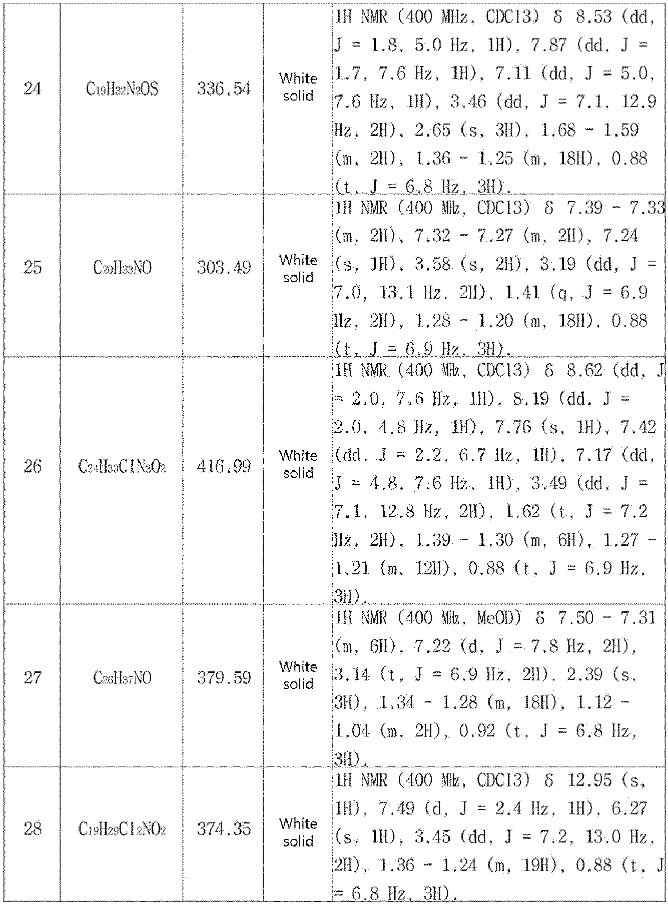

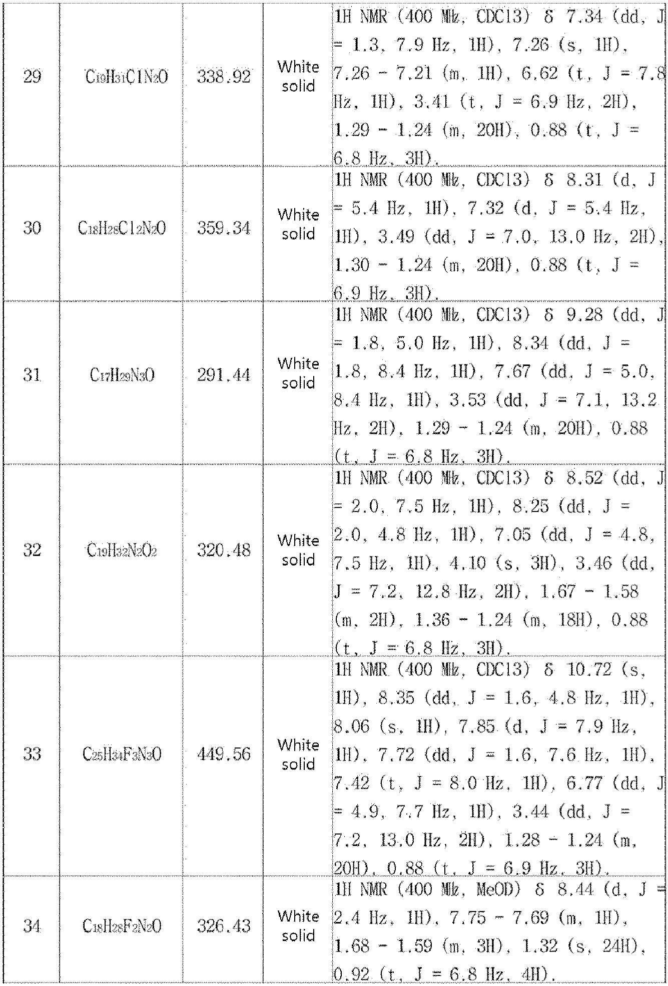

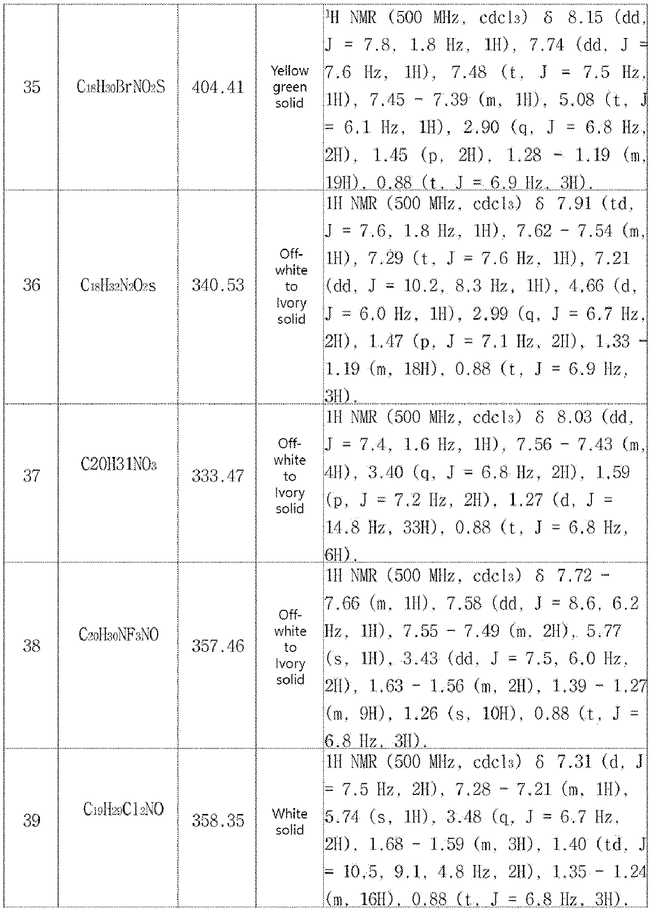

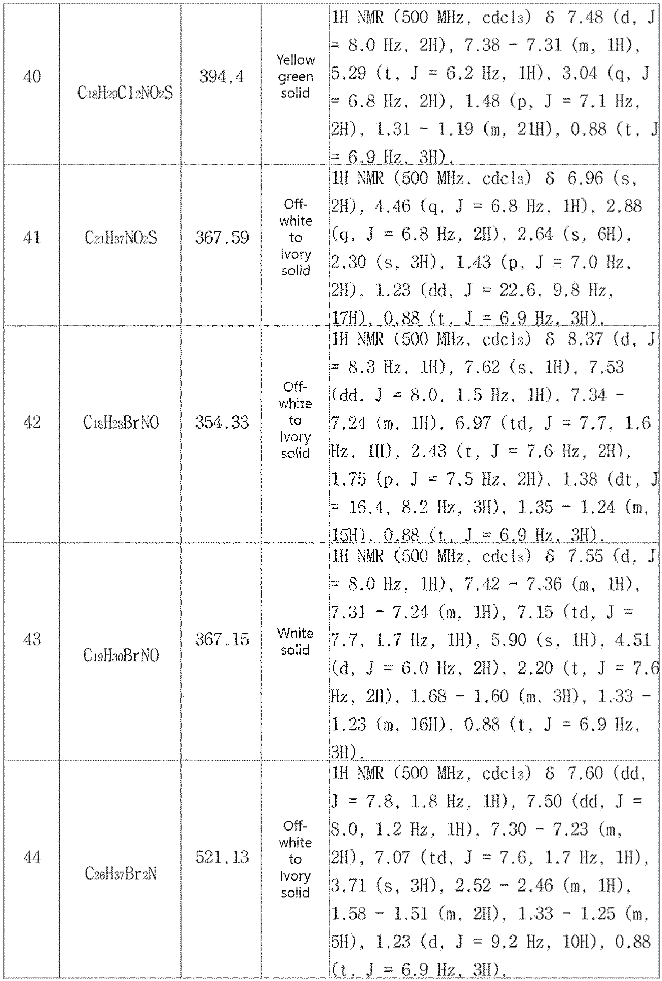

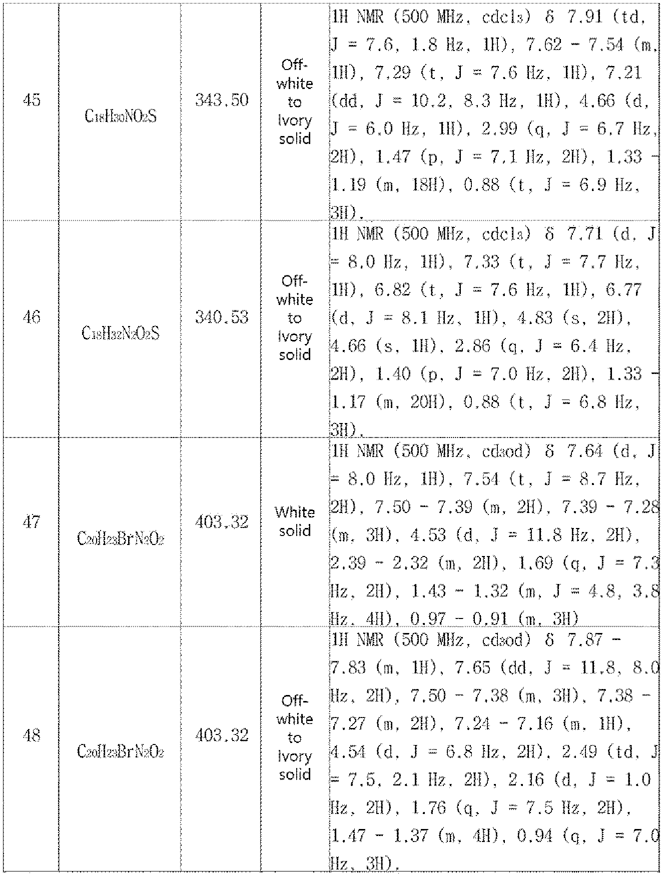

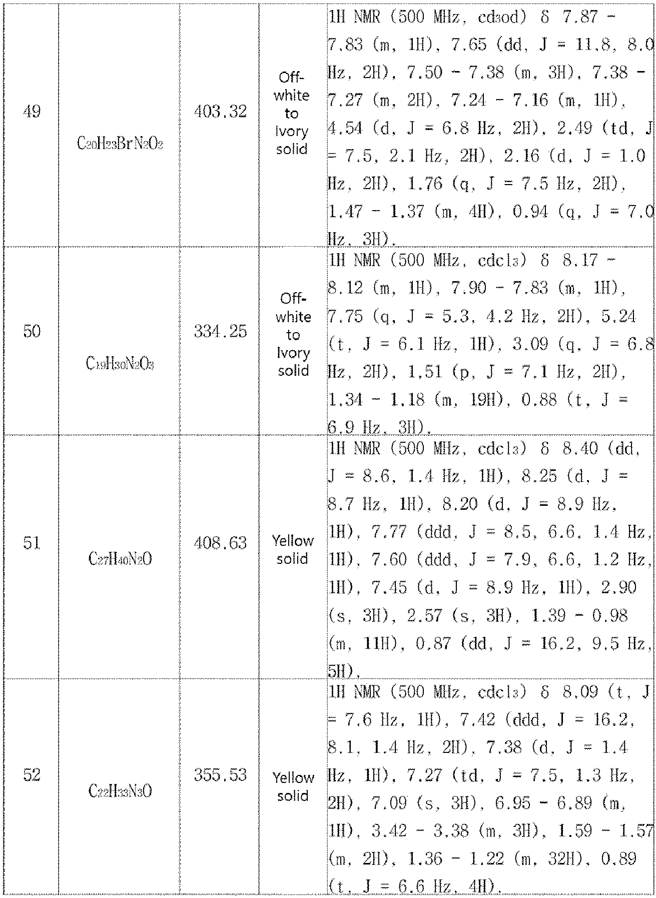

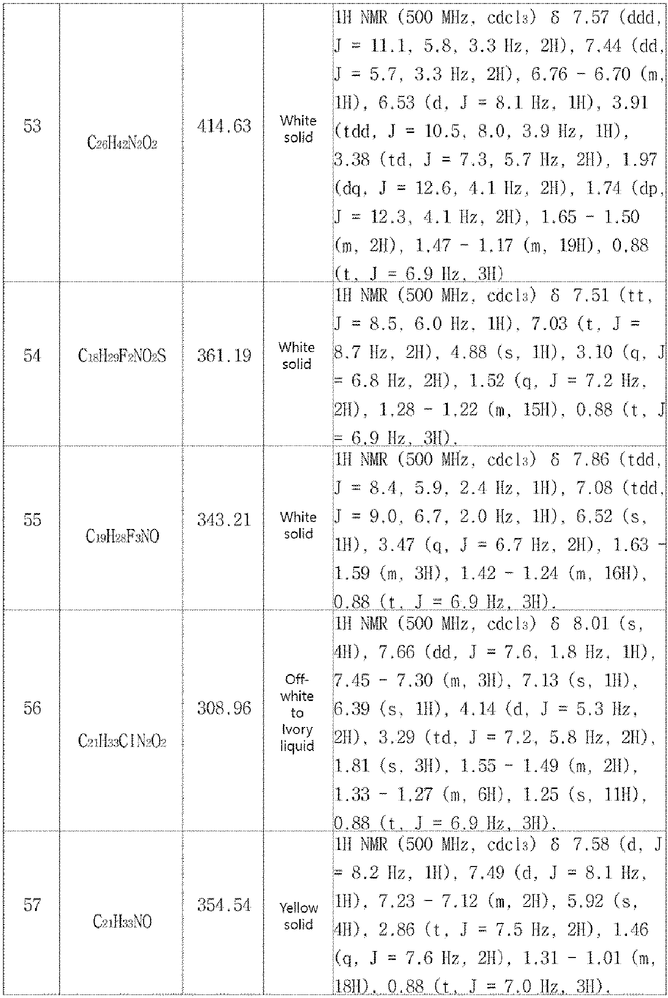

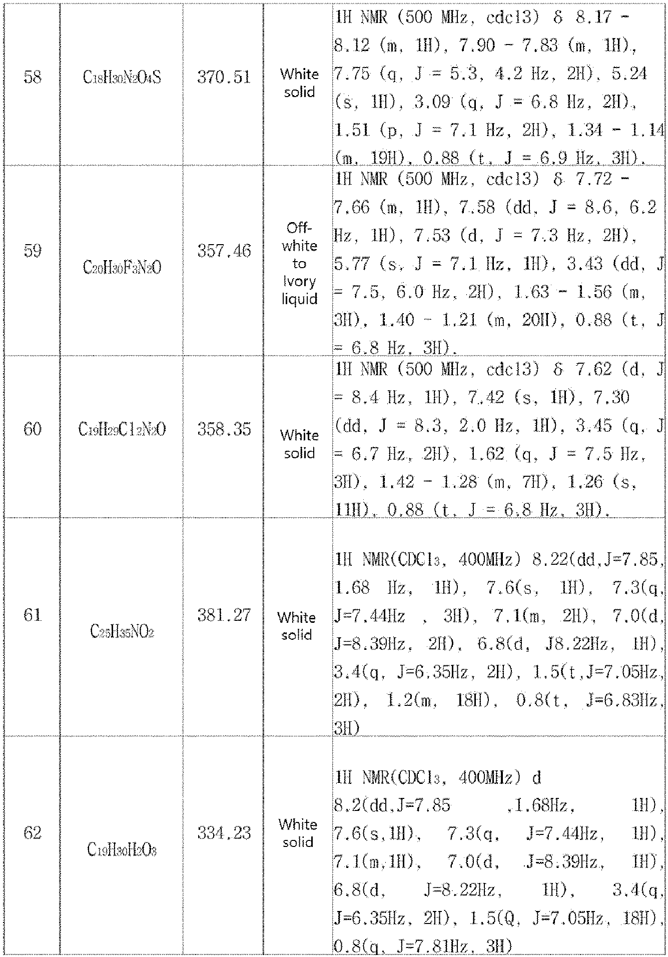

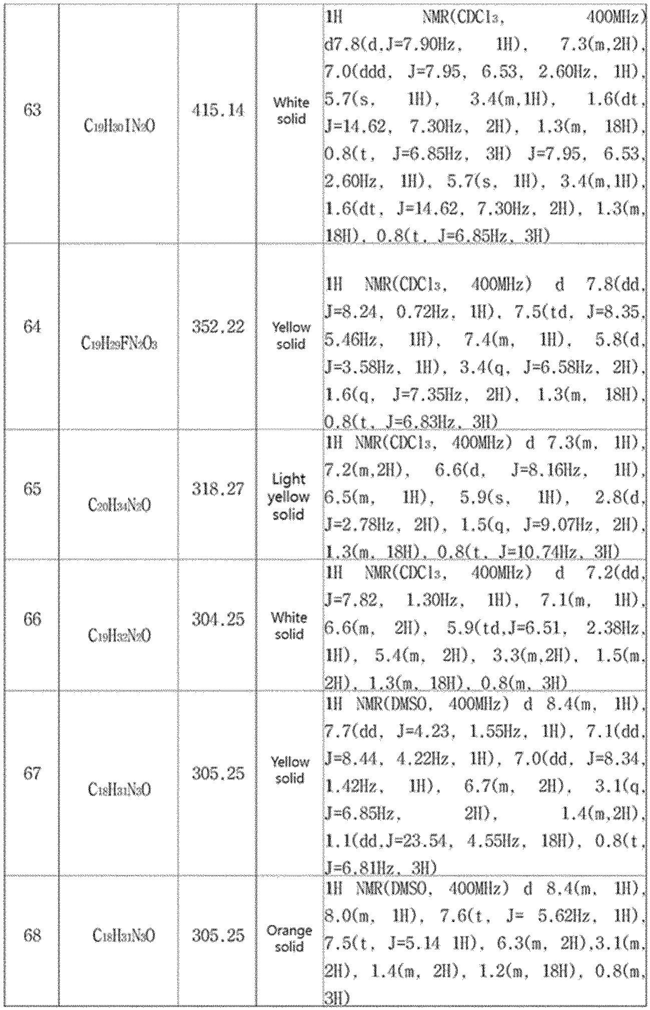

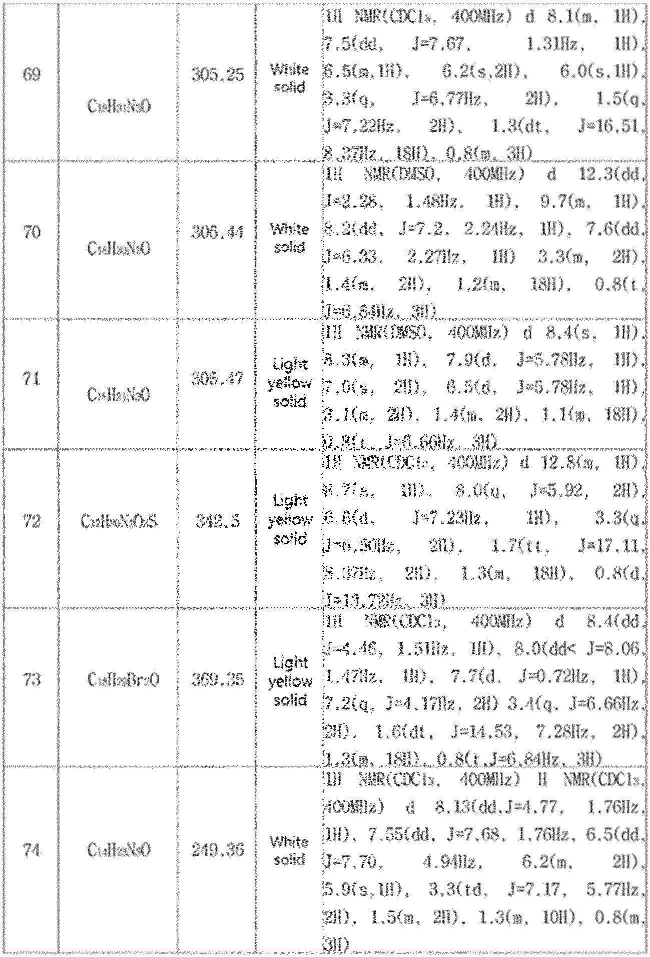

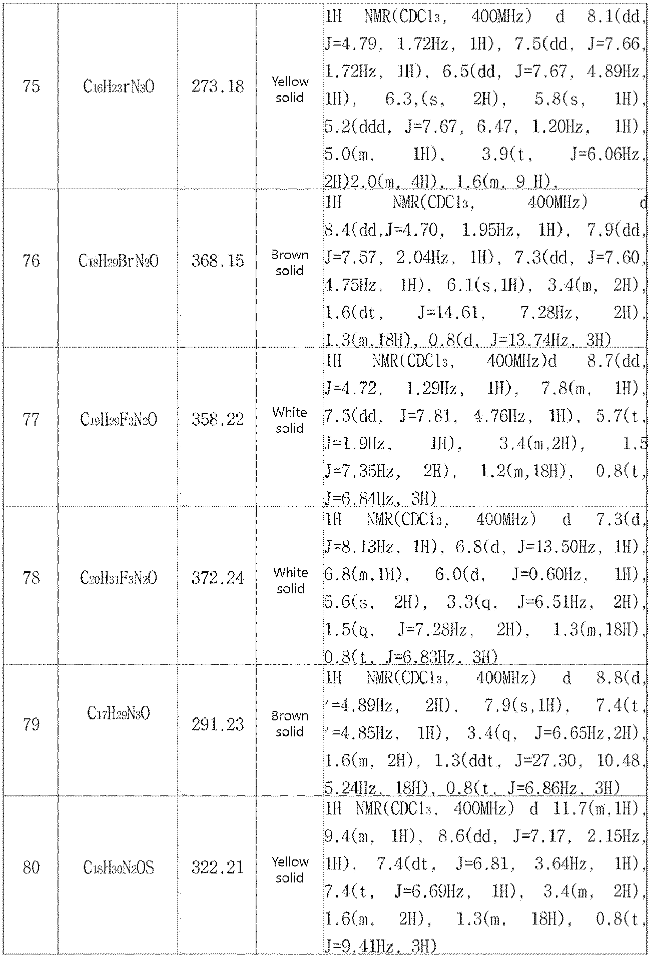

### Example 1: Validation of T Cell Proliferation Inhibition Effect

To investigate whether the compounds obtained in the Preparation Example have an inhibitory effect on T cell proliferation, the T cell proliferation was determined *ex vivo* using the fluorescent dye carboxyfluorescein diacetate succinimidyl ester (CFSE), which binds to intracellular molecules by covalent bonds.

Spleens were harvested and minced from C57BL/6 mice at 7 weeks of age, and then single cells were isolated using a strainer (40 µm pore size, SPL). Red blood cells were removed using ACK (Ammonium-Chloride-Potassium) lysis buffer to isolate only leukocytes. Subsequently, CD90.2 microbeads (130-121-278, Miltenyi Biotech.) were added, and subjected to react at 4°C for 20 minutes. After that, spleen T cells were isolated using a MACS Magnetic Stand and LS column. The isolated T cells from the spleen were resuspended in 1 mL of Free media (RPMI-1640 + 200 U/mL penicillin + 200 µg/mL streptomycin), followed by the addition of 0.3 µL of CFSE (10 mM), and reacted at 37°C for 5 minutes. Subsequently, 10 mL of Free media was added to stop the reaction, and the cells were centrifuged to obtain the cell pellet. RPMI 1640 medium containing 10% FBS, 100 U/mL penicillin, and 100 µg/mL streptomycin was added to suspend the cells, and 2×10⁵ cells per well were seeded into a 96-well plate and T cells were activated by treatment with CD3 and CD28 antibodies (each at 0.5 µg/mL).

The compound diluted in DMSO was treated in triplicate at concentrations of 10 µM, 30 µM, and 50 µM, respectively. After incubation at 37°C with 5% CO₂ for 3 days, cell proliferation levels were determined using a FACS Canto machine.

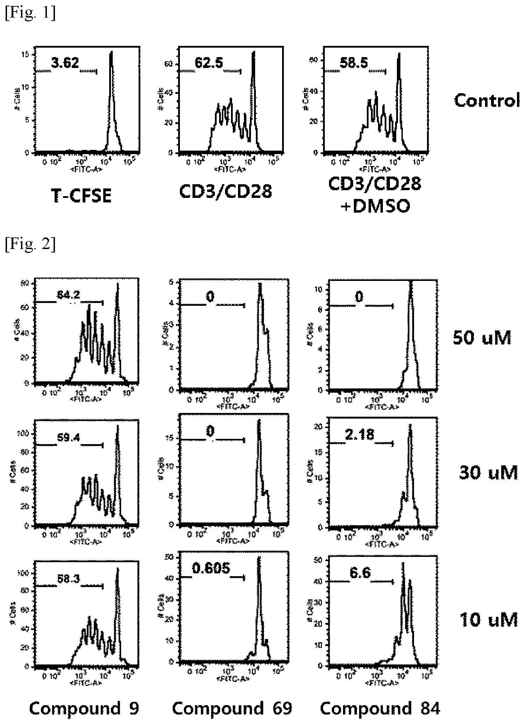

In the FACS graph of FIG. 1, the y-axis represents the quantity (number) of cells, while the x-axis represents the fluorescence level of the cells. Cells stained with CFSE exhibit a decrease in CFSE intensity as they undergo cell division. This reduction in CFSE fluorescence results in a leftward shift along the x-axis in the graph. This indicates cell proliferation.

FIG. 2 depicts the graphs of cell proliferation read by FACS after administration of Compounds 9, 69, and 84, respectively. Each graph shows increasing drug concentrations administered from bottom to top, corresponding to drug concentrations of 10 µM, 30 µM, and 50 µM, respectively. Compounds 69 and 84 demonstrate inhibition of cell proliferation at concentrations ranging from 10 to 30 µM. However, Compound 9 shows no inhibition of cell proliferation even at a concentration of 50 µM.

To collectively indicate the proliferation inhibition rate for the experimental results, the relative value of the proliferation amount after compound administration to the proliferation amount after CD3/CD28 + DMSO administration was calculated. Subsequently, it was converted into an inhibition amount and expressed as a percentage, thereby determining the proliferation inhibition rate. Specifically, the proliferation inhibition rate was calculated as follows: Proliferation inhibition rate (%) = {1-(Proliferation amount in drug-treated cells ÷ Proliferation amount after CD3/CD28+DMSO treatment)}×100

**[Table 2]**

| | Proliferation inhibition rate (%) | | |
|---|---|---|---|
| | 50 µM | 30 µM | 10 µM |
| Compound 10 | 92% | 72% | 15% |
| Compound 11 | 99% | 62% | 10% |
| Compound 12 | 99% | 38% | 5% |
| Compound 13 | 100% | 48% | 10% |
| Compound 14 | 100% | 100% | 100% |
| Compound 15 | 90% | 35% | 10% |
| Compound 16 | 100% | 100% | 0% |
| Compound 19 | 100% | 99% | 19% |
| Compound 25 | 99% | 90% | 20% |
| Compound 26 | 100% | 100% | 73% |
| Compound 27 | 100% | 96% | 62% |
| Compound 28 | 91% | 47% | 11% |
| Compound 29 | 65% | 33% | 5% |
| Compound 30 | 55% | 41% | 16% |
| Compound 31 | 69% | 57% | 15% |
| Compound 32 | 54% | 42% | 12% |
| Compound 33 | 100% | 100% | 33% |
| Compound 34 | 87% | 43% | 6% |
| Compound 35 | 93% | 92% | 85% |
| Compound 37 | 65% | 55% | 23% |
| Compound 38 | 85% | 73% | 40% |
| Compound 39 | 89% | 61% | 7% |
| Compound 40 | 97% | 97% | 86% |
| Compound 41 | 100% | 100% | 80% |
| Compound 43 | 72% | 50% | 27% |
| Compound 45 | 97% | 59% | 30% |
| Compound 46 | 93% | 94% | 47% |
| Compound 50 | 97% | 66% | 0% |
| Compound 51 | 100% | 100% | 100% |
| Compound 52 | 100% | 95% | 42% |
| Compound 53 | 85% | 65% | 22% |
| Compound 54 | 77% | 60% | 55% |
| Compound 55 | 95% | 82% | 52% |
| Compound 56 | 62% | 50% | 26% |
| Compound 57 | 100% | 100% | 93% |
| Compound 58 | 68% | 51% | 39% |
| Compound 60 | 98% | 72% | 10% |
| Compound 61 | 98% | 86% | 16% |
| Compound 63 | 89% | 78% | 30% |
| Compound 64 | 100% | 100% | 79% |
| Compound 65 | 77% | 43% | 39% |
| Compound 67 | 100% | 88% | 35% |
| Compound 68 | 100% | 67% | 57% |
| Compound 69 | 100% | 100% | 99% |
| Compound 70 | 98% | 78% | 29% |
| Compound 71 | 100% | 100% | 100% |
| Compound 72 | 86% | 80% | 68% |
| Compound 73 | 86% | 76% | 32% |
| Compound 74 | 95% | 91% | 66% |
| Compound 75 | 98% | 98% | 95% |
| Compound 76 | 65% | 49% | 36% |
| Compound 77 | 66% | 47% | 24% |
| Compound 78 | 66% | 45% | 8% |
| Compound 79 | 100% | 88% | 46% |
| Compound 80 | 100% | 100% | 43% |
| Compound 82 | 85% | 80% | 60% |
| Compound 83 | 100% | 63% | 0% |
| Compound 84 | 100% | 96% | 89% |

On the other hand, as shown in Table 3 below, compounds not belonging to Formula 1 according to the present invention were confirmed to have a very low proliferation inhibition rate.

**[Table 3]**

| | Proliferation inhibition rate (%) | | |
|---|---|---|---|
| | 50 µM | 30 µM | 10 µM |
| Compound 1 | 7% | 6% | 9% |
| Compound 2 | 11% | 10% | 11% |
| Compound 3 | 18% | 15% | 9% |
| Compound 4 | 16% | 12% | 23% |
| Compound 9 | 0% | 0% | 0% |
| Compound 10 | 3% | 9% | 5% |
| Compound 81 | 22% | 2% | 0% |

### Example 2: Cytotoxicity experiment

To evaluate the cytotoxicity of the compound according to the present invention, the following experiment was conducted.

The T lymphocyte cell line Jurkat E6-1, purchased from the Korean Cell Line Bank, was cultured in RPMI-1640 medium comprising 10% FBS, 100 U/mL penicillin, and 100 µg/mL streptomycin. After culturing, the cells were harvested and centrifuged at 1,300 rpm for 5 minutes. After removal of the supernatant, the cells were resuspended in the same medium at a concentration of approximately 2.2×10⁵ cells/mL, and 90 µL of the cell suspension was distributed per well into a 96-well plate (Costar 96 well cell culture plate, Coming). After treating 10 µL of each compound at different concentrations onto the respective wells of the plate, it was cultured for 24 hours at 37°C in a 5% CO₂ incubator. Subsequently, 10 µL of CCK-8 was added to each well, and it was cultured again for 2 hours at 37°C in a 5% CO₂ incubator. Absorbance was then measured at 450 nm to determine the relative cell survival rate compared to untreated cells, as shown in Table 4.

The values listed in Table 4 represent the relative cell survival rates in the groups treated with each compound at different concentrations, with the cell survival rate of untreated cells set as 100%.

**[Table 4]**

| | Relative cell survival rate (%) | | | |
|---|---|---|---|---|
| | 50 µM | 30 µM | 20 µM | 10 µM |
| Compound 10 | 83 | 85 | 88 | 91 |
| Compound 12 | 79 | 83 | 92 | 100 |
| Compound 13 | 57 | 70 | 71 | 95 |
| Compound 14 | 72 | 70 | 81 | 86 |
| Compound 16 | 85 | 92 | 101 | 121 |
| Compound 17 | 66 | 80 | 80 | 106 |
| Compound 18 | 99 | 99 | 101 | 102 |
| Compound 19 | 83 | 82 | 87 | 94 |
| Compound 20 | 105 | 107 | 108 | 113 |
| Compound 21 | 124 | 109 | 106 | 101 |
| Compound 22 | 36 | 39 | 42 | 42 |
| Compound 23 | 54 | 62 | 62 | 66 |
| Compound 24 | 54 | 61 | 68 | 71 |
| Compound 25 | 84 | 87 | 90 | 95 |
| Compound 26 | 82 | 85 | 97 | 101 |
| Compound 27 | 63 | 71 | 77 | 109 |
| Compound 29 | 60 | 77 | 80 | 107 |
| Compound 30 | 106 | 108 | 109 | 120 |
| Compound 31 | 102 | 102 | 111 | 111 |
| Compound 33 | 70 | 79 | 91 | 100 |
| Compound 34 | 87 | 101 | 109 | 112 |
| Compound 35 | 86 | 87 | 89 | 97 |
| Compound 37 | 92 | 90 | 90 | 90 |
| Compound 38 | 89 | 89 | 91 | 90 |
| Compound 39 | 79 | 78 | 102 | 89 |
| Compound 40 | 86 | 96 | 99 | 105 |
| Compound 41 | 80 | 82 | 83 | 85 |
| Compound 42 | 103 | 104 | 109 | 110 |
| Compound 43 | 108 | 108 | 104 | 101 |
| Compound 44 | 93 | 94 | 98 | 100 |
| Compound 45 | 74 | 76 | 94 | 96 |
| Compound 47 | 96 | 97 | 97 | 98 |
| Compound 48 | 98 | 99 | 99 | 100 |
| Compound 49 | 98 | 98 | 98 | 98 |
| Compound 50 | 68 | 70 | 75 | 74 |
| Compound 51 | 90 | 92 | 94 | 99 |
| Compound 52 | 84 | 84 | 85 | 88 |
| Compound 53 | 95 | 98 | 97 | 96 |
| Compound 54 | 76 | 77 | 83 | 89 |
| Compound 55 | 105 | 108 | 110 | 115 |
| Compound 56 | 86 | 89 | 91 | 92 |
| Compound 57 | 101 | 104 | 108 | 108 |
| Compound 58 | 94 | 97 | 98 | 100 |
| Compound 61 | 74 | 76 | 94 | 96 |
| Compound 63 | 79 | 75 | 78 | 70 |
| Compound 64 | 87 | 87 | 87 | 91 |
| Compound 65 | 77 | 86 | 84 | 87 |
| Compound 67 | 92 | 93 | 93 | 94 |
| Compound 68 | 82 | 88 | 91 | 92 |
| Compound 69 | 84 | 88 | 94 | 95 |
| Compound 70 | 125 | 124 | 122 | 119 |
| Compound 71 | 88 | 92 | 94 | 101 |
| Compound 73 | 104 | 98 | 97 | 94 |
| Compound 74 | 77 | 81 | 84 | 101 |
| Compound 75 | 85 | 86 | 95 | 97 |
| Compound 76 | 70 | 92 | 95 | 115 |
| Compound 77 | 123 | 123 | 124 | 116 |
| Compound 78 | 120 | 117 | 113 | 112 |
| Compound 81 | 108 | 107 | 105 | 103 |
| Compound 82 | 88 | 97 | 90 | 95 |
| Compound 83 | 77 | 87 | 105 | 102 |
| Compound 84 | 94 | 96 | 94 | 96 |

### Example 3: Confirmation of cytokine expression reduction effect

To confirm the effect of compounds of the present invention on the secretion of cytokines by T cells, the following experiment was conducted.

Spleens were harvested and minced from C57BL/6 mice at 7 weeks of age, and single cells were isolated using a strainer (40 µm pore size, SPL). Red blood cells were removed using ACK (Ammonium-Chloride-Potassium) lysis buffer to isolate only leukocytes. These were then reacted with 10 v/v% CD90.2 microbeads (130-121-278, Miltenyi Biotech.) at 4°C for 20 minutes, resulting in the isolation of spleen T cells from magnetic-based purification. The T cells were cultured in a 96-well plate at an amount of 2×10⁵ cells/well. For T cell activation, each well was treated with anti-CD3/CD28 antibodies at a concentration of 1 µg/mL. Subsequently, Vehicle (DMSO) and compounds (Compound 14, Compound 19, Compound 26, Compound 31, Compound 35, Compound 40, Compound 41, Compound 56, Compound 69, Compound 75) at a concentration of 30 µM were treated in each well, and it was cultured at 37°C in a 5% CO₂ incubator. After 24 and 72 hours, the T cell culture medium was taken, and centrifugation was performed at 13,000 rpm for 10 minutes to obtain the supernatant.

To measure the concentration of cytokines present in the T cell culture medium, Mouse Th1/Th2/Th17 Cytokine CBA Kit (Cytometric Bead Array kit) 560485 from BD Science was utilized for flow cytometry analysis. The secretion levels of IL-2 at 24 hours after each drug treatment, and IFN-γ, TNF-α, and IL-17A at 72 hours were measured after each drug treatment.

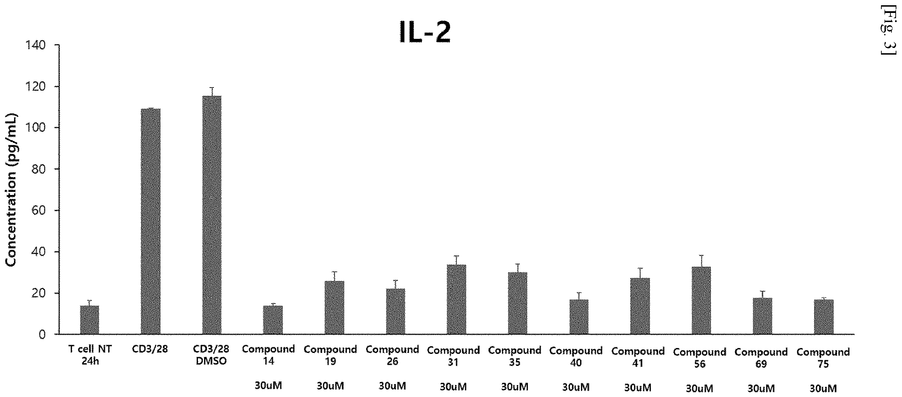

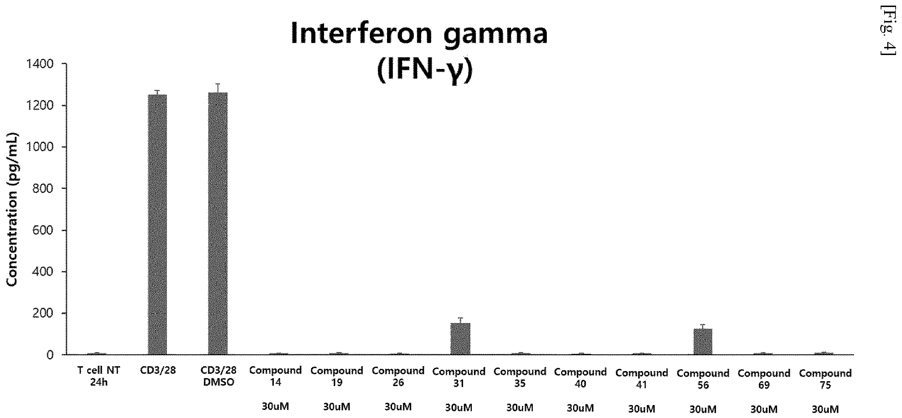

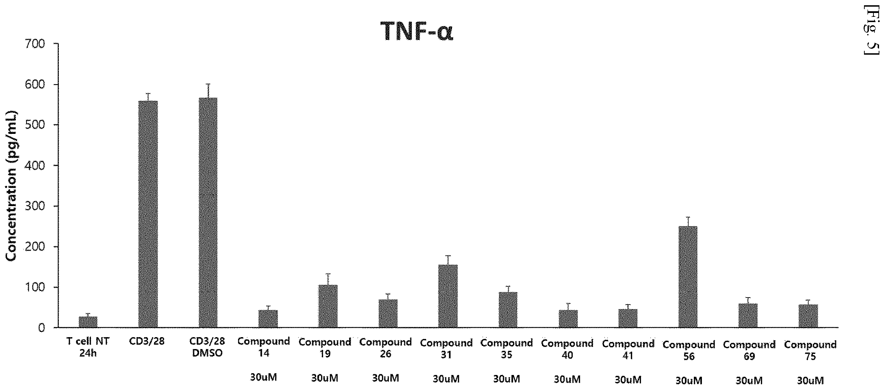

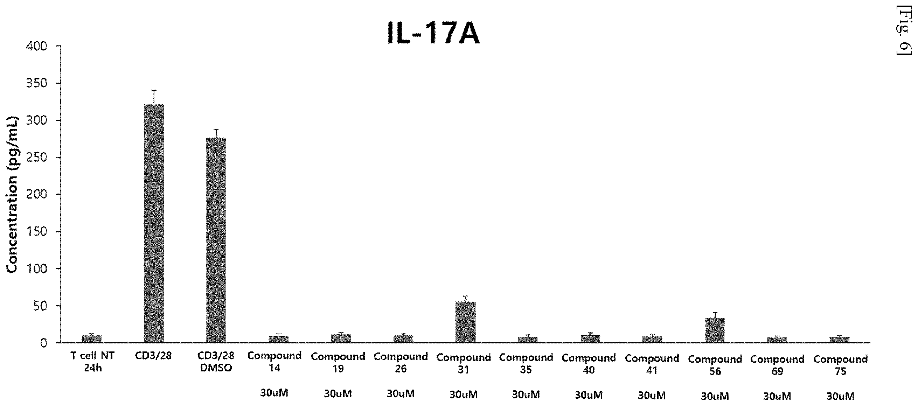

As shown in FIGs 3 to 6 representing the experimental results, T cells stimulated with anti-CD3/CD28 antibodies but not treated with compounds from the present invention exhibited abundant secretion of IL-2, IFN-γ, TNF-α, and IL-17A. In contrast, when treated with compounds (Compound 14, Compound 19, Compound 26, Compound 31, Compound 35, Compound 40, Compound 41, Compound 56, Compound 69, Compound 75), there was a significant decrease in the secretion of these cytokines. Based on this, it is evident that compounds of the present invention have the effect of reducing cytokine secretion in the process of inhibiting T cell proliferation.

### Example 4: Confirmation of treatment efficacy in Psoriasis model

To confirm the therapeutic effect of compounds according to the present invention on psoriasis, a psoriasis animal model was established to evaluate the alleviation of skin lesions and changes in immune cell profiles by administering Compound 69 or Compound 83 via injection.

To establish a psoriasis mouse animal model, BALB/c mice (7 weeks old, 20g, female) were purchased from Hana Biotech Co. and allowed to acclimate to the housing environment for one week. Afterward, the fur on the dorsal area of the mice was initially shaved using a shaver, followed by complete removal with depilatory cream (Nicrin Cream, comprising thioglycolic acid 80%, manufactured by ILDONG Pharmaceutical CO.,LTD). The mice were then housed for 24 hours to confirm any wounds on their backs.

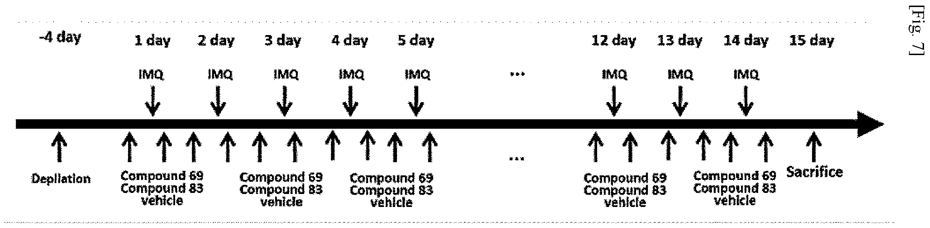

Subsequently, in order to verify the preventive and therapeutic effects of compounds according to the present invention on psoriasis, five experimental groups were established as shown in FIG. 7 and Table 5. From the second to the fifth group, Aldara^{®} cream was applied once daily for two weeks (14 days, total of 14 applications) at a dose of 80 mg (imiquimod, 4 mg) per mouse to induce psoriasis. Furthermore, to compare the preventive and therapeutic effects of compounds according to the present invention (Compound 69 and Compound 83) on psoriasis, 40 mg/kg of the compound per mouse was dissolved in DMSO corresponding to 10% of the dosing volume, and then diluted in a mixture of Cremophor EL-PEG 400-distilled water to achieve a final ratio of DMSO:Cremophor EL:PEG 400:distilled water to 1:1:4:4 (v/v/v/v). Subsequently, the respective injections were intraperitoneally administered at a dose of 100 µL per mouse, twice a day with a 12-hour interval. For the Vehicle control group, injections excluding the drug but comprising the vehicle solution (DMSO:Cremophor EL:PEG 400:distilled water = 1:1:4:4 (v/v/v/v)) were administered at a dose of 100 µL per mouse, twice a day with a 12-hour interval.

**[Table 5]**

| **Group** | **Topical application of imiquimod 4 mg** | **Intervention** | **No. of Mouse** |
|---|---|---|---|
| **Normal** | X | Depilation only (no administration) | 5 |
| **Paoriasis** | O | Depilation only (no administration) | 5 |
| **Psoriasis (Vehicle)** | O | Solvent (DMSO:Cremophor EL:PEG 400:distilled water = 1: 1:4:4 (v/v/v/v)) 100 µL, administered intraperitoneally twice daily | 5 |
| **Psoriasis (Compound 69)** | O | Solvent (DMSO:Cremophor EL:PEG 400:distilled water = 1: 1:4:4 v/v/v/v) 100 µL + Compound 40 mg/kg, administered intraperitoneally twice daily | 5 |
| **Psoriasis (Compound 83)** | O | | 5 |

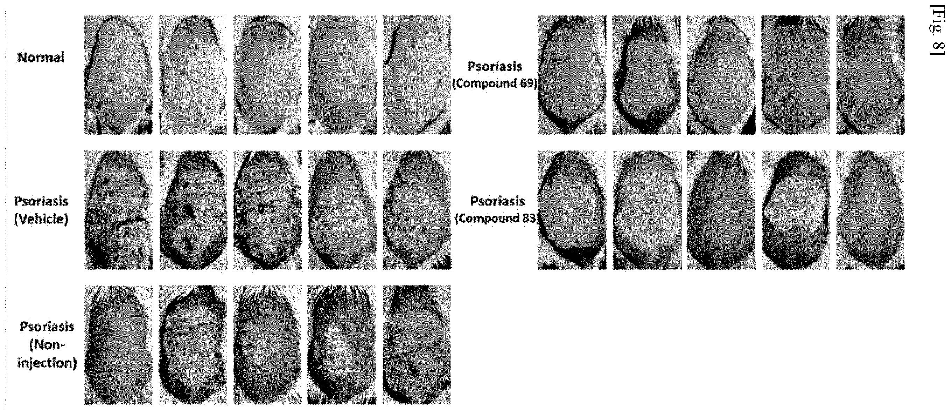

14 days after the experiment, the erythema, thickness, and exfoliation of the skin on the back of each mouse were observed in the respective groups, and the pathological tissue of the area was analyzed through H&E staining. As shown in FIG. 8, in the groups where only psoriasis was induced (Psoriasis, Non-injection) and in the groups where psoriasis was induced along with intraperitoneal injection of vehicle (Psoriasis, Vehicle), erythema, thickness, and exfoliation of the skin area were severe. However, in the groups treated with Compound 69 or Compound 83, skin lesions were either minimally present or hardly observed.

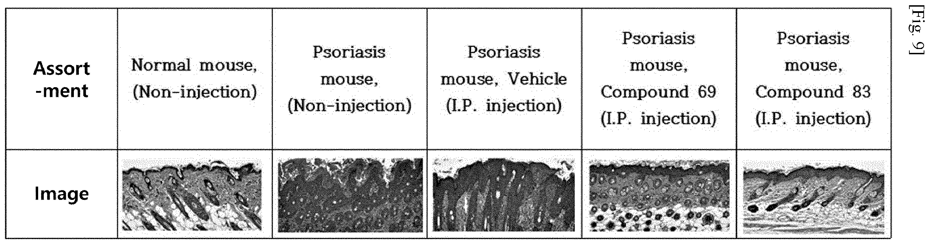

As shown in FIG. 9, in the groups where only psoriasis was induced (Psoriasis, Non-injection) and in the groups where psoriasis was induced along with intraperitoneal injection of vehicle (Psoriasis, Vehicle), severe hyperkeratosis, parakeratosis, and irregular acanthosis were observed, along with significant infiltration of inflammatory cells. However, in the groups treated with Compound 69 or Compound 83, such phenomena were either minimally present or hardly observed.

To assess the impact of each compound on immune cells *in vivo,* the spleens of mice treated with each compound were harvested, and cells were extracted, and cells were suspended in MACS buffer (2 mM EDTA, 0.5% FBS, 137 mM NaCl, 2.7 mM KCl, 10 mM Na₂HPO₄, 1.8 mM KH₂PO₄, Miltenyi Biotech.). For flow cytometry analysis, each mouse's splenocytes were aliquoted at 5×10⁵ cells per FACS tube (5 mL round tube, Falcon). To prevent non-specific antibody binding, each tube was treated with 1 µg/mL of anti-mouse CD16/CD32 (*InVivo*MAb) for 30 minutes, followed by centrifugation at 13,000 rpm for 5 minutes, and resuspended in 100 µL of MACS buffer. Subsequent experiments were conducted depending on the type of immune cells to be examined.

For B cells, each FACS tube was stained with 1 µg/mL of FITC-conjugated anti-B220 antibody (eBioscience^{™}) at 4°C for 30 minutes, followed by washing with 2 mL of MACS buffer. Subsequently, each tube was centrifuged at 15,000 rpm for 5 minutes, the supernatant was removed, and the precipitated cells were fixed using 200 µL of 4% paraformaldehyde solution.

For T cells, each FACS tube was stained with 1 µg/mL of PerCP-Cy5.5-conjugated anti-CD3 antibody (Biolegend), FITC-conjugated anti-CD8 antibody (eBioscience^{™}) and Cy7-conjugated anti-CD4 antibody (eBioscience^{™}) at 4°C for 30 minutes, followed by washing with 2 mL of MACS buffer. Subsequently, each tube was centrifuged at 15,000 rpm for 5 minutes, the supernatant was removed, and the precipitated cells were fixed using 200 µL of 4% paraformaldehyde solution.

For macrophages, each FACS tube was stained with 1 µg/mL of FITC-conjugated anti-CD11b antibody (eBioscience^{™}) at 4°C for 30 minutes, followed by washing with 2 mL of MACS buffer. Subsequently, each tube was centrifuged at 15,000 rpm for 5 minutes, the supernatant was removed, and the precipitated cells were fixed using 200 µL of 4% paraformaldehyde solution.

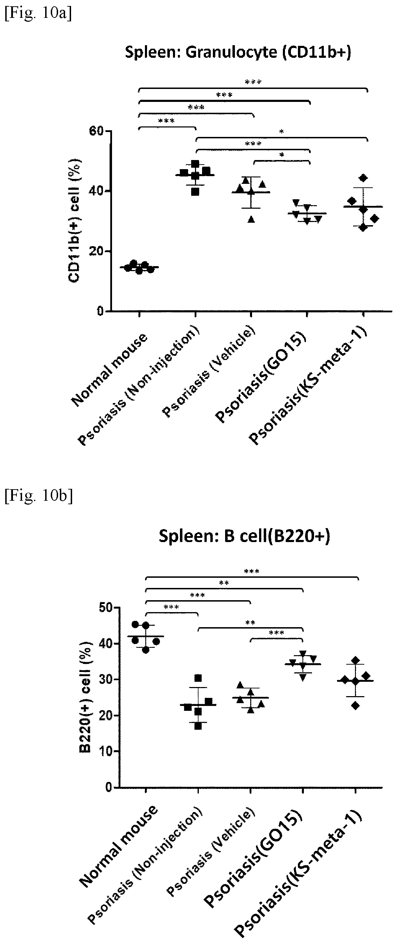

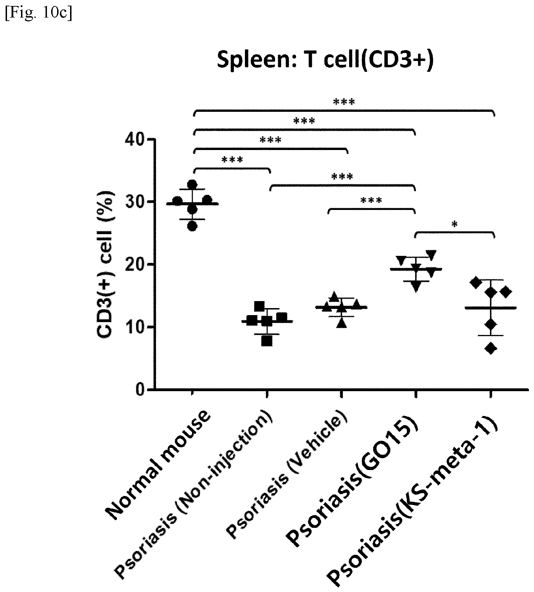

Through the process of staining immune cells and conducting flow cytometric analysis, as seen in FIGs 10a to 10c, in the groups where psoriasis was induced by applying imiquimod only (Psoriasis, Non-injection) and in the groups where psoriasis was induced by applying imiquimod along with intraperitoneal injection of Vehicle (Psoriasis, Vehicle), there was a sharp increase in CD1 1b-positive granulocytes and a relative decrease in the composition ratio of B cells and T cells in the spleen. However, in the groups treated with Compound 69 or Compound 83 via intraperitoneal injection, the proportion of CD11b granulocytes decreased, accompanied by an increase in the proportion of B cells and T cells. Consequently, it was confirmed that when the effective compound is administered, the composition ratio of CD11b-positive granulocytes, B cells, and T cells in the spleen beccomes similar to that observed in normal mice, indicating the therapeutic efficacy of the drugs.

Through the above results, it has been confirmed that Compound 69 or Compound 83, compounds according to the present invention, exhibits therapeutic efficacy in the psoriasis, autoimmune disease model and affects the functioning of immune cells.

## Claims

1. A compound represented by Formula 1, its stereoisomer, solvate, hydrate, crystalline form, or pharmaceutically acceptable salt: 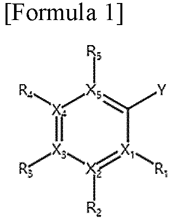 in the Formula 1,
- among X₁, X₂, X₃, X₄ and X₅, one or two atoms are nitrogen, and the other atoms are carbon,
- each R₁, R₂, R₃, R₄ and R₅ is independently selected among hydrogen, halogen, C1-C14 alkyl, C2-C14 alkenyl, C2-C14 alkynyl, C5-C14 aryl, C5-C14 arylalkyl, C3-C14 cycloalkyl, C3-C14 cycloalkylalkyl, C3-C14 heteroaryl, C3-C14 heteroarylalkyl, C3-C14 heterocycloalkyl, C3-C14 heterocycloalkylalkyl, C1-C14 alkoxy, C5-C14 aryloxy, C5-C14 heteroaryloxy, -NO₂, -NR₆, -SO₂R₆, -SO₂N(R₆)₂, -CF₃, -OH, cyanide, thiol, triazine, triazole, tetrazole, thiazole, diazole, imidazole, alkylamine, cycloalkylamine, heterocycloamine, and biotin, and
- Y is selected among -OH, thiol, phenoxy, cyanide, thiazole, diazole, imidazole, triazine, triazole, tetrazole, , -CON(R₆)₂, -NR₆COR₆, -SO₂N(R₆)₂, -NH₂, -NO₂, -COOR₆, - R₇COOR₆, -R₇CON(R₆)₂, -CONHSR₆, -CONHR₇SR₆, -CONHSSR₆, -CONHR₇SSR₆, - CONHR₇SO₂R₆, -CONHOR₆, -CONHR₇OR₆, -CON(R₇)₂CON(R₆)₂, and - CON(R₇)₂NR₇COR₆,
wherein each R₆ and R₇ is independently selected among hydrogen, C1-C14 alkyl, C2-C14 alkenyl, C2-C14 alkynyl, C6-C12 aryl, C6-C12 arylalkyl, C3-C12 cycloalkyl, C3-C12 cycloalkylalkyl, C3-10 heteroaryl, C3-10 heteroarylalkyl, C3-10 heterocycloalkyl, C3-10 heterocycloalkylalkyl, alkylamine, cycloalkylamine, heterocycloamine and biotin, unsubstituted or substituted with one or more of hydrogen, halogen, thiol, phenoxy, cyanide, thiazole, diazole, imidazole, triazine, triazole, tetrazole, C1-C6 alkyl, C2-C6 alkenyl, C2-C6 alkynyl, C6-C12 aryl, C6-C12 arylalkyl, C3-C12 cycloalkyl, C3-C12 cycloalkylalkyl, C3-10 heteroaryl, C3-10 heteroarylalkyl, C3-10 heterocycloalkyl, C3-10 heterocycloalkylalkyl, C1-C6 alkoxy, -CF₃, amine, C1-6 alkylamine, and -CN.

2. A composition comprising the compound represented by Formula 1, its stereoisomer, solvate, hydrate, crystalline form, or pharmaceutically acceptable salt according to claim 1.

3. The composition according to claim 2, wherein the composition is a pharmaceutical composition for preventing or treating immune disorders selected from autoimmune diseases, transplant rejections, allergic diseases, and inflammatory diseases.

4. The composition according to claim 2, wherein the composition is a food composition for preventing or alleviating immune disorders selected from autoimmune diseases, transplant rejections, allergic diseases, and inflammatory diseases.

5. The composition according to claim 2, wherein the composition is a cosmetic composition for preventing or alleviating immune disorders selected from autoimmune diseases, transplant rejections, allergic diseases, and inflammatory diseases.
